# EUROPEAN PATENT APPLICATION

(11) **EP 1 813 367 A2**
(43) Date of publication of application: **01.08.2007**
(21) Application number: 07100804.9
(22) Date of filing: 19.01.2007
(51) Int. Cl.: B22F 3/22

(54) **Metal injection molded article with a radiopaque dispersion and methods of making same**

(30) Priority: 27.01.2006 US 342182
(71) Applicant: Accellent, Inc., Collegeville, PA 19426 (US)
(72) Inventor: Broadley, Mark W, Downingtown PA Pennsylvania 19335 (US); Eckert, John, Boyertown PA Pennsylvania 19512 (US); Farina, Jeffrey M, Zionsville PA Pennsylvania 18092 (US)
(74) Representative: McIlroy, Steven David

(57) **Abstract**

The present invention includes a method of making a metal injection molded article and the metal injection molded article. The method includes mixing metal powders and a binder to form a mixture. The metal powders include a bulk material and at least one radiopaque material. The mixture is injected into a mold and processed into a green part. The green part is debound to form a brown part. The brown part is sintered to create a completed article. The completed article includes a metal alloy that includes the bulk material and a dispersion of the at least one radiopaque material substantially independent of the metal alloy.

## Description

### Field of Invention

The material and methods disclosed herein relate to a metal injection molded article, and a metal injection molded article with a radiopaque dispersion.

### Background of Invention

Metal Injection Molding ("MIM") has been practiced for years in industries such as agricultural, automotive, business machine, food & beverage, hardware, medical, small appliance, and sporting good. MIM provides articles for those industries, as well as others, that have strengths that are comparable to articles produced by forging, stamping, casting, and machining processes, while, at the same time, providing the ability to produce high volumes of the articles in a cost effective manner. Additional advantages of MIM include the ability to produce articles with intricate shapes, the ability to produce articles with close tolerances, and the ability to produce articles with multiple components in one mold, thereby reducing assembly costs.

Because of the materials typically used in metal injection molding, MIM articles generally have low radiopacity, which raises concerns in many applications. Low radiopacity is of particular concern where the article is a medical device, and more particularly where the article is a medical device that is intended to be implanted or used in a patient. When a medical device with low radiopacity is placed in a patient, it is difficult to view the device with imaging technologies such as x-ray fluoroscopy. As a result, proper placement and/or alignment of the medical device cannot be readily verified.

Attempts have been made to overcome this problem. For example, it is common practice to attach radiopaque markers to a medical device. The markers are generally made from materials such as iridium, platinum, gold, and tantalum. The markers, because of their size and composition, may cause galvanic corrosion. Another disadvantage of the markers is that they are generally not integral with the medical device. Therefore, the markers can inadvertently dislodge from the medical device or otherwise interfere with the performance and/or deployment of the medical device. Yet another disadvantage of the markers is that they are generally not co-extensive with the entire surface of the medical device. Consequently, imaging technology, such as x-ray fluoroscopy, will show only a portion of the medical device.

It is also common practice to coat or plate medical devices with radiopaque materials. The resulting radiopaque coated or plated medical devices suffer from similar disadvantages as medical devices with radiopaque markers, i.e., galvanic corrosion, interference with performance of the medical device, interference with deployment of the medical device, and inadvertent separation of the coating or plating.

What is needed is an article that combines the benefits of metal injection molded articles with the benefits of high radiopacity, while, at the same time, overcoming the problems associated with the prior art.

### Summary of Invention

One embodiment of the invention encompasses a metal injection molded article that includes a metal alloy and a dispersion of at least one radiopaque material substantially independent of the metal alloy.

Another embodiment of the invention includes a method of making a metal injection molded article. The method includes mixing metal powders and a binder to form a mixture. The metal powders include a bulk material and at least one radiopaque material. The mixture is injected into a mold and processed into a green part. The green part is debound to form a brown part. The brown part is sintered to create a completed article. The completed article includes a metal alloy that includes the bulk material and a dispersion of the at least one radiopaque material substantially independent of the metal alloy.

In a further embodiment of the invention, the completed article comprises a metal alloy that includes both the bulk material and at least one radiopaque material dissolved in the alloy.

### Brief Description of Drawings

For the purpose of illustrating the invention there is shown in the drawings various forms which are presently preferred; it being understood, however, that this invention is not limited to the precise arrangements and instrumentalities particularly shown.

FIG. 1 is a perspective view of an article according to one embodiment of the invention.

FIG. 1A is an exaggerated view of a portion of the article of FIG. 1.

FIG. 2 is a perspective view of an article according to a second embodiment of the invention.

FIG. 3 is a perspective view of an article according to a third embodiment of the invention.

FIG. 4 is a perspective view of an article according to a fourth embodiment of the invention.

### Detailed Description of the Illustrated Embodiments

The present invention includes a method of making a metal injection molded article. The method includes mixing metal powders and a binder to form a mixture. The metal powders include a bulk material and at least one radiopaque material. The mixture is injected into a mold and processed into a green part. The green part is debound to form a brown part. The debinding includes removing a substantial portion of the binder from the mold. The brown part is sintered to create a completed article. The completed article includes a metal alloy comprising the bulk material. The completed article also includes a dispersion of the at least one radiopaque material, which is substantially independent of the metal alloy.

The bulk material can be powders of any metal suitable for metal injection molding. Preferably, the bulk material includes fine metal powders. More preferably, the bulk material includes fine metal powders having a diameter of less than 50 microns. Most preferably, the bulk material includes fine metal powders having a diameter of less than 25 microns.

The bulk material preferably includes powders capable of forming various metals and alloys. More particularly, the bulk material can include powders capable of forming any metals and alloys suitable for the medical industry. For example, the bulk material can include powders capable of forming the following medical metals and alloys: 304 stainless steel per ASTM F899, 316L stainless steel per ASTM F899 and ASTM F138, commercially pure Ti per ASTM F67, Ti 6A1 4V per ASTM F1472, Ti 6A1 4V ELI per ASTM F136, Nitinol per ASTM F2063, Cobalt Chrome Moly per ASTM F75, and/or combinations thereof.

The bulk material can include powders capable of forming an implantable austenitic stainless steel alloy (e.g., 316L stainless steel per ASTM F138). An austenitic alloy formed from the bulk material generally has excellent corrosion resistance, excellent elongation and is easily weldable. Generally such an alloy is used where corrosion resistance is more important than strength or wear resistance.

The bulk material can include powders capable of forming a precipitation hardened stainless steel alloy (e.g., 17-4PH stainless steel per ASTM F899). This material exhibits a good balance between corrosion resistance and strength. The strength and hardness can be modified by adjusting the temperature at which the material is heat treated after sintering. The precipitation hardened alloy generally provides for better corrosion resistance than 400 series stainless steels and better strength than 300 series stainless steels.

The bulk material can include powders capable of forming a martensitic stainless steel (e.g. 440C per ASTM F899). This material exhibits the highest hardness and strength among the stainless steels with some tradeoff in corrosion resistance.

The bulk material can include a combination of low alloy steel, carbon, nickel, and molybdenum. The combination provides for a multi-purpose, economical material for non medical applications that provides flexibility in various properties such as strength, hardness, and wear resistance. The material can be plated or coated for additional corrosion resistance.

The radiopaque material can be any material that precludes x-rays or other types of radiation commonly used in diagnostic imaging from penetrating it. When the radiopaque material is present in the completed article, it essentially blocks radiation from penetrating the completed article where it is present. The blocking of radiation is advantageous in many fields, but particularly in the medical device field. A medical device having a radiopaque material integral with the structural component of the device allows the device to be visible when viewed by imaging technology such as x-ray fluoroscopy.

The radiopaque material preferably comprises at least one element and/or at least one alloy. If the radiopaque material comprises at least one element, preferably the one or more element(s) is selected from the group consisting of iridium, platinum, gold, rhenium, tungsten, palladium, rhodium, tantalum, silver, ruthenium, and hafnium. If the radiopaque material comprises at least one alloy, preferably the one or more alloy(s) includes one or more of iridium, platinum, gold, rhenium, tungsten, palladium, rhodium, tantalum, silver, ruthenium, and hafnium.

The binder can include any material commonly used as a binder in metal injection molding. Preferably, the binder is a polymer binder. The binder may also be a combination of two or more polymeric materials.

The bulk material, radiopaque material, and the binder are mixed together to form a mixture. Preferably, the mixture is flowable at relatively low temperature and pressure. A flowable mixture allows the mixture to fill all of the crevices and small dimensional features of a mold.

The binder, the bulk material, and the at least one radiopaque material are mixed using a conventional mixing apparatus. The temperature at which the materials are mixed can vary.

The mixture is injected into a mold. Generally, the mixture is injected with an injection molding machine. Injection molding machines are known in the art and are typically capable of applying from less than one hundred to several hundred tons of force to a mold. The mold is typically constructed with internal cooling passages to solidify the flowable mixture prior to removal from the mold. The mold cavity typically is larger than that of the desired finished part to account for the shrinkage that occurs after binder removal. The mold structure can be a rigid or a flexible material. The mold typically includes vents or bleeder lines to allow air to escape from the mold during the molding process. Alternatively, the mold can include a porous metal or ceramic insert to allow air to escape from the mold.

After the mixture is injected into the mold, the mixture is processed Processing of the mixture includes applying pressure to the mold/mixture to form a green part. The applied pressure is typically about 10-12 ksi.

After molding, the green part is removed from the mold and an initial debinding step may be performed. During initial debinding, some portion of the binder is removed from the green part to create a brown part. The binder can be removed by heating the green part, generally with a low-temperature thermal treatment. When the green part is heated, a portion of the binder will melt, decompose, and/or evaporate. The binder can also be removed by solvent or water extraction or by a combination of heating and solvent extraction.

After initial debinding, the brown part is thermally debound and sintered to create a completed article. Sintering is the process of heating metal powders until the powders adhere to each other, thereby forming a substantially solid form having the same or nearly the same mechanical properties as the metal in its cast or wrought form. Sintering can include heating the brown part to a temperature close to, but not exceeding, the melting point of the part. Where it is desirable for the radiopaque material to remain generally independent of the alloy formed by the bulk material, the radiopaque material is selected to have limited solubility in the bulk material.

Preferably, the sintering temperature is about 20°C to about 100°C less than the melting point of the bulk material. That temperature is then maintained for a set period of time. Under those conditions, interparticulate melting and substantial diffusion can occur, thereby eliminating interstitial void spaces and causing the material densities to become substantially solid. Where it is desirable for the radiopaque material to remain generally independent of the alloy formed by the bulk material, the sintering temperature and the length of time of sintering should be limited so as to limit diffusion of the radiopaque material. Specific times and temperatures are dependent on the characteristics of the bulk material, the radiopaque material, and so on.

Complete solidification (i.e., 100% density) is desired but generally does not occur. Preferably, the density of the completed article is at least 95% of theoretical. More preferably, the density of the completed article is at least 97% of theoretical. These density measurement percentages are based on the theoretical density of the article.

During the sintering steps, the brown parts typically shrink due to the decrease in the size of the interstitial void spaces. The shrinkage typically causes the completed article to be about 10% to about 30% smaller than the green part from which it is made. More particularly, the completed article is about 20% smaller than the green part. The amount of shrinkage should be considered when designing/selecting a mold for a particular part.

In another embodiment of the present invention, the at least one radiopaque material forms an alloy with the bulk material. The alloying of the radiopaque material can be achieved during the sintering process by exposing the brown part to a sufficient time and temperature to insure long range diffusion of the radiopaque material.

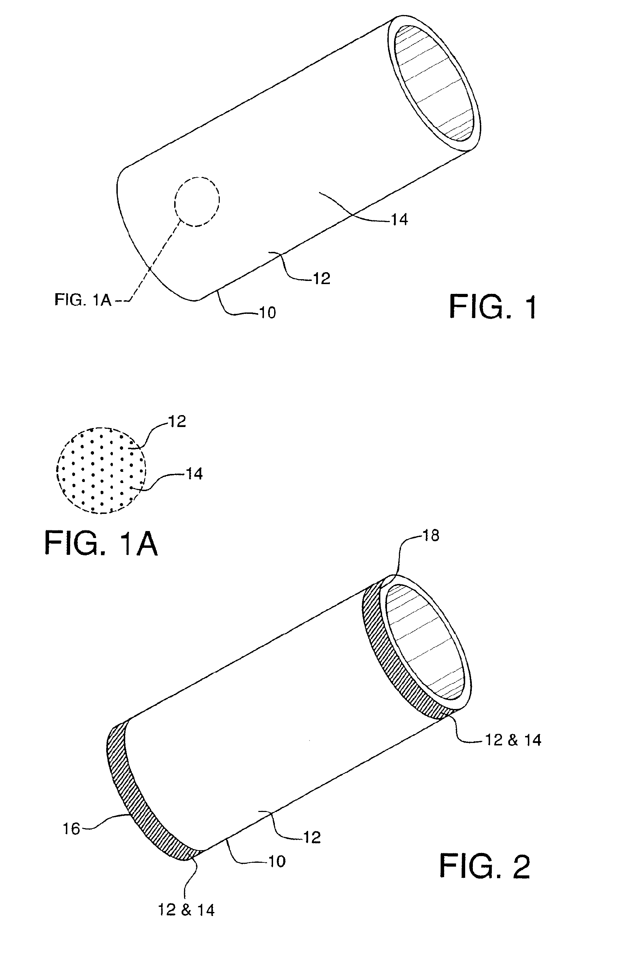

As shown in Figure 1, the article made by a method of the present invention is an implantable medical device 10. The device 10 includes a metal alloy 12 and a dispersion of at least one radiopaque material 14 substantially independent of the metal alloy. The dispersion of the radiopaque material 14 is interspersed homogenously throughout the metal alloy 12. FIG. 1A shows a portion of the device 10 at an exaggerated scale so as to illustrate the homogenous dispersion. This exaggerated scale is presented merely for illustrative purposes. Preferably, the dispersion of the radiopaque material 14 is not visible to the naked eye.

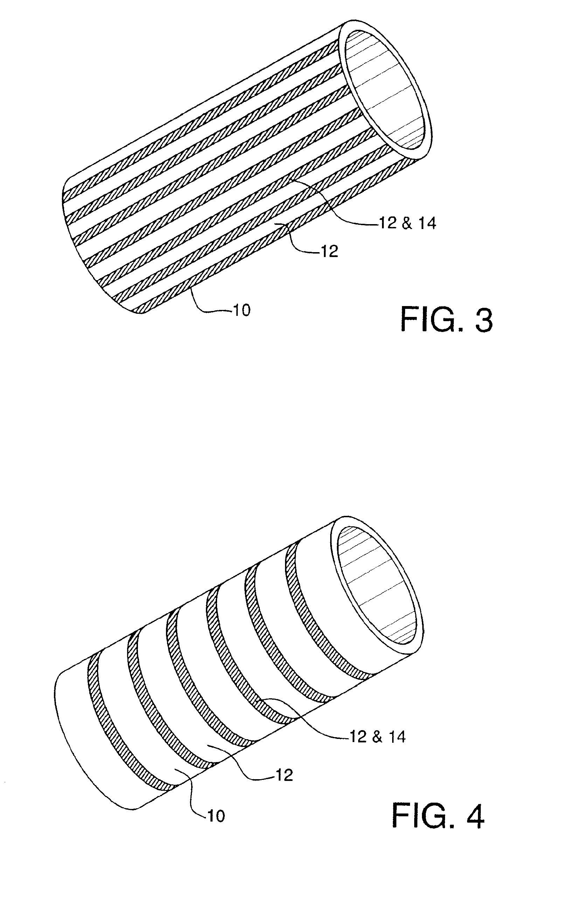

Alternatively, the dispersion 14 can be positioned at specific locations on the article. For example, the dispersion 14 can be homogenously mixed with the metal alloy at the ends 16, 18 of the article only (as shown in FIG. 2). It can be homogeneously mixed with the metal alloy along only longitudinal lines traversing the length of the article (as shown in FIG. 3). It can be homogeneously mixed with the metal alloy along only latitudinal lines traversing the circumference of the article (as shown in FIG. 4). The alternative orientations for the dispersion 14 can be accomplished by configuring the mold such that the radiopaque material mixes with the metal alloy in only discrete locations (e.g., at the end of the article). Although FIGS. 2 - 4 illustrate the dispersion of the radiopaque material 14 as being visible with the naked eye, this is done merely for illustrative purposes. Preferably, the dispersion of the radiopaque material 14 is not visible to the naked eye.

While the article is shown as an implantable medical device, the article is not so limited. The article can be all or just a portion of any structure having the elements described herein. The article can be all or a part of a product including, but not limited to, actuators, automotive components, cellular telephones, dental instruments, electrical connectors, electronic heat sinks and hermetic packages, fiber optic connectors, hard disk drives, hydraulic line couplings, pharmaceutical devices, power hand tools, and sporting equipment. The article is particularly advantageous in the medical field where it can be, for example, in the form of laparoscopic parts such as graspers, dissectors, and surgical instrument end effectors. Preferably, the article is used in the medical field as a medical device including, but not limited to, stents, grafts (e.g., aortic grafts), artificial heart valves, cerebro-spinal fluid shunts, pacemaker components, axius coronary shunts, catheters, biopsy sectioning and retrieval equipment, vena cava filters, reconstructive implants such as intramedullary nails and screws, fixation plates and anchors, orthopedic implants such as knee, hip and shoulder components, spinal prostheses, and dental implants.

Where device 10 is a stent, it can be placed into a patient to treat a variety of medical conditions. The most common use of stents is to hold open clogged blood vessels after angioplasty. When used for this purpose a catheter is moved through the body to the site where the blood vessel is blocked. A balloon is then inflated to open the vessel. In some cases, the stent is then placed to decrease chances that the blood vessel will close up again.

Stents also are used to hold open blood vessels, bile ducts, or other pathways in the body that have been narrowed or blocked by tumors or other obstructions. Areas where stents are most often used for this reason include: the esophagus, to treat blockages or narrowing of the esophagus that make it difficult to swallow; the bile ducts in the pancreas or liver, when an obstruction prevents bile from draining into the digestive tract; and the airways of the lungs, to treat obstructions that interfere with normal breathing.

To place the stent, a medical personnel makes a very small incision in the skin, about the size of a pencil tip. The stent, which is placed on the end of a catheter, is threaded under guidance of imaging technology such as x-rays to the area of treatment. Because imaging technology is generally used to guide the placement of the stent, it is advantageous to have a catheter and a stent with high radiopacity so that they can be more easily viewed by the medical personnel performing the placement. High radiopacity is similarly advantageous on other types of medical devices that rely on imaging technology to guide the placement. Once in place, the high radiopacity allows medical personnel to view the device to ensure proper alignment, potential damage to adjacent tissue, and so on.

Completed articles produced by the MIM process can be further processed. For example, the completed articles can be machined to provide a smooth surface or other similar characteristics. The completed article can be coated to enhance functionality. The completed article can be annealed to relieve internal stresses or soften the metal.

An article made by metal injection molding provides the design flexibility of plastic injection molding (e.g., the ability to produce articles with intricate shapes, the ability to produce articles with close tolerances, and the ability to produce articles with multiple components in one mold) with the material properties at or near that of wrought metals.

Although the disclosure describes the metal injection molded article as a medical device, the present invention is not so limited. The article can be all or part of any product where a metal injection molded article having high radiopacity is desired.

It will be appreciated by those skilled in the art that the present invention may be practiced in various alternate forms and configurations. The previously detailed description of the disclosed methods is presented for clarity of understanding only, and no unnecessary limitations should be implied therefrom.

## Claims

1. A metal injection molded article comprising:
a metal alloy; and
a dispersion of at least one radiopaque material substantially independent of the metal alloy.

2. A metal injection molded article according to claim 1, wherein the article comprises a medical device.

3. A metal injection molded article according to claim 1 or 2, wherein the metal alloy comprises one or more of commercially pure Titanium, Ti 6A1 4V, Ti 6A1 4V ELI, Nitinol, Cobalt Chrome Moly, 316L stainless steel, 304 stainless steel, and 17-4PH stainless steel.

4. A metal injection molded article according to any of claims 1 to 3, wherein the at least one radiopaque material comprises at least one radiopaque element.

5. A metal injection molded article according to claim 4, wherein the at least one radiopaque element is selected from the group consisting of iridium, platinum, gold, rhenium, tungsten, palladium, rhodium, tantalum, silver, ruthenium, and hafnium.

6. A metal injection molded article according to any of claims 1 to 5, wherein the at least one radiopaque material comprises at least one radiopaque alloy.

7. A metal injection molded article according to claim 6, wherein the at least one radiopaque alloy comprises one or more of the iridium, platinum, gold, rhenium, tungsten, palladium, rhodium, tantalum, silver, ruthenium, and hafnium.

8. A metal injection molded article according to any of claims 1 to 7, wherein the article has a density of at least 95%.

9. A metal injection molded article according to claim 8, wherein the article has a density of at least 97%.

10. A method for making a metal injection molded article, the method comprising:
mixing metal powders and a binder to form a mixture, the metal powders comprising a bulk material and at least one radiopaque material;
injecting the mixture into a mold;
processing the mixture in the mold to form a green part;
debinding the green part to form a brown part; and
sintering the brown part to create a completed article.

11. A method according to claim 10, wherein the binder comprises a polymer binder.

12. A method according to claim 10 or 11, wherein the debinding step comprises heating the green part.

13. A method according to any of claims 10 to 12, wherein the debinding step comprises dissolving the binder in at least one solvent or water.

14. A method according to any of claims 10 to 13, wherein the completed article has a density of at least 95%.

15. A method according to claim 14, wherein the completed article has a density of at least 97%.

16. A method according to any of claims 10 to 15, wherein the radiopaque material is selected to have limited solubility in the matrix material during sintering.

17. A method according to claim 16, wherein the completed article comprising a metal alloy comprising the bulk material and a dispersion of the at least one radiopaque material substantially independent of the metal alloy.

18. A method according to any of claims 10 to 16, wherein the sintering step is performed at a temperature at or above the alloying temperature of the at least one radiopaque material.

19. A method according to claim 18, wherein the completed article comprising a metal alloy comprising the bulk material alloyed with a dispersion of the at least one radiopaque material.

20. A method according to any of claims 10 to 19, wherein the at least one radiopaque material comprises at least one radiopaque element.

21. A method according to claim 20, wherein the at least one radiopaque element is selected from the group consisting of iridium, platinum, gold, rhenium, tungsten, palladium, rhodium, tantalum, silver, ruthenium, and hafnium.

22. A method according to any of claims 10 to 21, wherein the at least one radiopaque material comprises at least one radiopaque alloy.

23. A method according to claim 22, wherein the at least one radiopaque alloy comprises one or more of the iridium, platinum, gold, rhenium, tungsten, palladium, rhodium, tantalum, silver, ruthenium, and hafnium.

24. A method according to any of claims 10 to 23, further comprising machining the completed article.

25. A method according to any of claims 10 to 24, further comprising coating the completed article.

26. A method according to any of claims 10 to 25, further comprising annealing the completed article.

27. A method according to any of claims 10 to 26, where the article is a medical device.
